(19) **Europäisches Patentamt / European Patent Office / Office européen des brevets**

(11) **EP 2 422 813 A1**

(12) # EUROPEAN PATENT APPLICATION
published in accordance with Art. 153(4) EPC

(43) Date of publication:
**29.02.2012 Bulletin 2012/09**

(21) Application number: **10767094.5**

(22) Date of filing: **21.04.2010**

(51) Int Cl.:
*A61K 45/00* (2006.01)    *A23L 1/30* (2006.01)
*A61K 36/18* (2006.01)    *A61K 36/60* (2006.01)
*A61P 1/14* (2006.01)    *A61P 11/02* (2006.01)
*A61P 35/00* (2006.01)    *A61P 37/04* (2006.01)
*A61P 37/08* (2006.01)    *A61P 43/00* (2006.01)

(86) International application number:
**PCT/JP2010/057092**

(87) International publication number:
**WO 2010/123037 (28.10.2010 Gazette 2010/43)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.04.2009 JP 2009104316**
**12.05.2009 JP 2009115563**

(71) Applicant: **FUJIFILM Corporation**
**Tokyo 106-0031 (JP)**

(72) Inventors:
• **ODA, Yuriko.**
  **Kanagawa (JP)**
• **UEDA, Fumitaka.**
  **Kanagawa (JP)**

(74) Representative: **HOFFMANN EITLE**
**Patent- und Rechtsanwälte**
**Arabellastraße 4**
**81925 München (DE)**

(54) **Agent for regulating composition ratio of intestinal bacterial flora**

(57)    A food or pharmaceutical capable of regulating an intestinal bacterial flora distribution ratio, which is safe and has few side effects, is provided.

An intestinal bacterial flora distribution ratio regulator containing at least one sugar absorption-suppressing substance, and a food or pharmaceutical containing the same.

EP 2 422 813 A1

**Description**

Technical Field

The present invention relates to an intestinal bacterial flora distribution ratio regulator containing a sugar absorption-suppressing substance.

Background Art

According to recent studies, it has been revealed that, in human, one who is fat is in a state where the distribution ratio of bacteria belonging to *Bacteroidetes* is low and the distribution ratio of bacteria belonging to *Firmicutes* is high but, as the body weight reduces, the distribution ratio of *Bacteroidetes* increases and, to the contrary, the distribution ratio of *Firmicutes* decreases. Moreover, it has been found that one who is thin has a high distribution ratio of *Bacteroidetes* and a low distribution ratio of *Firmicutes* as compared with one who is fat (see Non-Patent Document 1).

Furthermore, when intestinal bacterial flora of a fat mouse and intestinal bacterial flora of a usual-weight mouse are transplanted to two groups of mice having no intestinal bacteria, respectively, it has been revealed that the body weight of the mice of the group to which the intestinal bacteria of the fat mouse is transplanted is significantly more than the body weight of the group to which the intestinal bacterial flora of the usual-weight mouse is transplanted (see Non-Patent Document 2).

Namely, by changing the distribution ratio of intestinal bacteria, one's body constitution itself is changed, and there is an effect of improving one's physical condition.

The sum of *Firmicutes* and *Bacteroidetes* reaches close to 90% of whole intestinal bacterial flora. It is difficult to exert such a large influence on bacteria by an oral substance.

As a method for proliferating *Bacteroidetes,* there is a report on a method of ingesting a fractooligosaccharide (FOS) and a certain effect can be expected (see Non-Patent Document 3).

However, in order to have the effect by the ingestion of the fractooligosaccharide, a large-amount ingestion is necessary and it is a physical burden to ingest it through meals or functional foods. Moreover, an oral substance having effects of proliferating bacteria belonging to *Bacteroidetes* and also decreasing bacteria belonging to *Firmicutes* has been unknown.

Background Art Documents

Non-Patent Documents

Non-Patent Document 1: Ley, RE., Turnbaugh, PJ., Klein, S., and Gordon, JI., Microbial ecology: human gut microbes associated with obesity. Nature, 444, 1022-3 (2006)

Non-Patent Document 2: Turnbaugh, PJ., Ley, RE., Mahowald, MA., Magrini, V., Mardis, ER., and Gordon, JI., An obesity-associated gut microbiome with increased capacity for energy harvest. Nature, 444, 1027-31 (2006)

Non-Patent Document 3: Nakanishi, Y, Murashima, K., Ohara, H., Suzuki, T., Hayashi, H., Sakamoto, M., Fukasawa, T., Kubota, H., Hosono, A., Kono, T., Kaminogawa, S., Benno, Y., Increase in terminal restriction fragments of Bacteroidetes-derived 16S rRNA genes after administration of short-chain fructooligosaccharides. Appl. Environ. Microbiol., 72, 6271-6 (2006)

Summary of the Invention

Problems that the Invention is to Solve

A problem that the invention is to solve is to provide a food or pharmaceutical capable of regulating an intestinal bacterial flora distribution ratio, which is safe and has few side effects.

Means for Solving the Problems

The present inventors have first found out that a certain kind of an oral substance has an action of proliferating bacteria of *Bacteroidetes* and decreasing bacteria of *Firmicutes*.

The invention is specifically composed of the following constitution.

(1) An intestinal bacterial flora distribution ratio regulator comprising at least one sugar absorption-suppressing substance.

(2) The intestinal bacterial flora distribution ratio regulator according to (1) above, wherein the sugar absorption-suppressing substance is an $\alpha$-glucosidase activity-inhibiting substance.

(3) The intestinal bacterial flora distribution ratio regulator according to (1) or (2) above, wherein the sugar absorption-suppressing substance contains at least one or more selected from the group consisting of plants belongint to the genus *Salacia,* mulberry leaves, Gymnema sylvestre, and ingredients contained in extracts thereof.

(4) The intestinal bacterial flora distribution ratio regulator according to any one of (1) to (3) above, wherein the sugar absorption-suppressing substance exhibits an activity of a sucrase 50% inhibitory concentration of 10 $\mu$g/ml to 10,000 $\mu$g/ml.

(5) The intestinal bacterial flora distribution ratio regulator according to any one of (1) to (4) above, wherein the sugar absorption-suppressing substance is at least one selected from plants belonging to the genus *Salacia* selected from *Salacia reticulate, Salacia oblonga,* and *Salacia chinensis* and ingredients contained in extracts thereof.

(6) The intestinal bacterial flora distribution ratio regulator according to any one of (1) to (5) above, wherein the intestinal bacterial flora distribution ratio regulation increases a ratio of *Bacteroidetes* and decreases a ratio of *Firmicutes* in intestinal bacterial flora.

(7) A food or pharmaceutical containing the intestinal bacterial flora distribution ratio regulator according to any one of (1) to (6) above.

Advantage of the Invention

According to the invention, there is provided an intestinal bacterial flora distribution ratio regulator, which is safe and has few side effects even when taken for a long period of time, and a food or pharmaceutical. By taking the same, intestinal bacterial flora balance is regulated and thus, slimming, improvement in body constitution, alleviation of allergy symptoms, improvement of immune indices, and the like are expected.

Mode for Carrying Out the Invention

The intestinal bacterial flora distribution ratio regulator of the invention contains at least one sugar absorption-suppressing substance.

The intestinal bacterial flora distribution ratio regulator of the invention has an action of changing a ratio of bacteria which live in the intestines. Specifically, it is an agent having an action of increasing bacteria of *Bacteroidetes* and decreasing bacteria of *Firmicutes.*

<Sugar absorption-suppressing substance>

The sugar absorption-suppressing substance is preferably an $\alpha$-glucosidase activity-inhibiting substance.

The $\alpha$-glucosidase activity-inhibiting substance is a substance which inhibits sugar metabolism by inhibiting an $\alpha$-glucosidase activity, and the sucrase 50% inhibitory concentration ($IC_{50}$ value) is preferably 0.01 $\mu$g/ml to 10,000 $\mu$g/ml, more preferably 0.1 $\mu$g/ml to 800 $\mu$g/ml, further preferably 0.5 $\mu$g/ml to 600 $\mu$g/ml, and particularly preferably 10 $\mu$g/ml to 450 $\mu$g/ml. When the inhibitory activity falls within the range, an action of suppressing glucose absorption from the digestive tracts and also abdomen enlarged feeling and gas generation can be suppressed. The sucrase 50% inhibitory concentration of the $\alpha$-glucosidase activity-inhibiting substance is more preferably 10 $\mu$g/ml to 5,000 $\mu$g/ml and further preferably 10 $\mu$g/ml to 2,000 $\mu$g/ml.

The sucrase 50% inhibitory concentration ($IC_{50}$ value) is measured by the following method.

[Experimental Method 1]

Measurement of sucrase $IC_{50}$ value

Preparation of sample solution: Two milligrams of a sample ($\alpha$-glucosidase activity-inhibiting substance) is weighed into a tube and 2 mL of water was added thereto, followed by thoroughly suspending the sample to form a sample solution having a concentration of 1 mg/mL. It is diluted with water so as to form solutions of 0, 50, 100, 250, and 500 $\mu$g/mL, respectively.

Preparation of substrate solution: Sucrose is dissolved in a 0.2M maleate buffer (pH 6.0) so as to be a sucrose concentration of 100 mM and the solution is used as a substrate solution.

Preparation of crude enzyme solution: After 1 g of intestinal acetone powder rat (manufactured by SIGMA) is suspended in 10 mL of physiological saline, the suspension is subjected to centrifugation (3,000 rpm, 4°C, 5 main). The resulting supernatant is separated and used as a crude enzyme solution.

To 500 µL of the aforementioned sample solution having each concentration was added 400 µL of the substrate solution, and the whole was pre-heated in a water bath at 37°C for 5 minutes. Thereto was added 100 µL of the crude enzyme solution, and reaction was performed at 37°C for 60 minutes. After completion of the reaction, the enzyme was deactivated by heating at 95°C for 2 minutes to terminate the reaction. The concentration of glucose formed is quantitatively determined using a commercially available kit/mutarotase-glucose oxidase method (Glucose CII Test Wako, Wako Pure Chemical Industries, Ltd.).

Preparation of blank: To 250 µl of the aforementioned sample solution having each concentration, 200 µL of the substrate solution and 50 µL of the crude enzyme solution are added, and the enzyme is immediately heat-deactivated by heating at 95°C for 2 minutes to obtain blank data.

A calibration curve is formed based on the obtained values and concentration at which the enzyme activity is inhibited by 50% ($IC_{50}$ value) is determined.

In the case of setting an intake or a standard intake per day of the α-glucosidase activity-inhibiting substance contained in the intestinal bacterial flora distribution ratio regulator, the amount of the α-glucosidase activity-inhibiting substance in the daily intake of the intestinal bacterial flora distribution ratio regulator is, for example, when an α-glucosidase activity-inhibiting substance having an $IC_{50}$ value of 50 µg/ml is used, preferably 10 to 600 mg, more preferably 40 to 450 mg, and particularly preferably 50 to 350 mg.

The amount of the α-glucosidase activity-inhibiting substance contained in the intestinal bacterial flora distribution ratio regulator can be properly calculated based on the above preferable amount per day. For example, in the case of preparing a tablet whose daily intake is three tablets, it is preferred to contain one third of the daily intake per tablet. Namely, the tablet contains the α-glucosidase activity-inhibiting substance in an amount of preferably 3 to 200 mg, more preferably 13 to 150 mg, particularly preferably 17 to 117 mg.

Moreover, the value determined by the following expression 1 is preferably 0.5 or more, more preferably 0.8 or more, and particularly preferably 1.7 or more.

The $IC_{50}$ value as a whole intestinal bacterial flora distribution ratio regulator is preferably 0.1 to 7.5, more preferably 0.15 to 4.50, and particularly preferably 0.3 to 3.75 as the value determined by the expression 2.

[Expression 1]

[Expression 1]

$$\text{α-Glucosidase activity-inhibiting substance in daily intake of intestinal bacterial flora distribution ratio regulator (mg)}/IC_{50} \text{ value (µg/ml)}$$

[Expression 2]

$$\text{Intestinal bacterial flora distribution ratio regulator (mg)}/IC_{50} \text{ value (µg/ml)}$$

The sugar absorption-suppressing substance is preferably plants belonging to the genus *Salacia,* mulberry leaves, Gymnema sylvestre, Du zhong leaves, guava, luobuma fiber, extracts of fermented black beans, tea leaves, magnolia bark, Artemisiae capillaris herba, Ctrus unshiu peel, evodia fruit, Japanese mint, Chinese bellflower, Chinese wolfberry fruit, schisandra fruit, etc., and extracts thereof, and more preferably, plants belonging to the genus *Salacia,* mulberry leaves, Gymnema sylvestre, and extracts thereof More preferably, extracts of plants belonging to the genus *Salacia,* extracts of mulberry leaves, or ingredients contained in Gymnema sylvestre may be mentioned.

<Ground product or extract of plants belonging to the genus *Salacia*>

The sugar absorption-suppressing substance is preferably a plant belonging to the genus *Salacia.* The plant belonging to the genus *Salacia* is usually contained as a ground product or an extract.

The plant belonging to the genus *Salacia* is a plant of Celastraceae which grows wild mainly in Sri Lanka, India, and Southeast Asia area. More specifically, one or more kinds of plants selected from *Salacia reticulate, Salacia oblonga, Salacia prinoides, Salacia chinensis, Salacia latifolia, Salacia burunoniana, Salacia grandiflora,* and *Salacia macrosperma* are used, and preferred is at least one plant selected from *Salacia reticulate, Salacia oblonga,* and *Salacia chinensis.*

The ground product or extract of the plant belonging to the genus *Salacia* means a ground product, a dried product, an extract, a dried powder thereof (extract powder), or the like of edible parts such as roots, trunks, leaves, flowers, and fruits. It is also suitable to mix and use one or more kinds of the parts. More preferably, an extract powder extracted from roots and trunks are used.

The extract powder is a dried product of one obtained by solvent extraction from the aforementioned edible parts and the like. The extraction solvent may be selected from the group consisting of water or alcohols such as methanol and ethanol, or mixed solvent of water and alcohols or ketones such as acetone. Preferably, water, an alcohol, or an aqueous alcohol is used. More preferably, as the extraction solvent, hot water or ethanol or an aqueous ethanol is used. Regarding the alcohol concentration of the above aqueous alcohol, one having a concentration of 30 to 90% by mass, preferably 40 to 70% by mass may be used.

As the drying method, spray drying, freeze-drying, and the like may be mentioned without limitation thereto.

<Other Ingredients>

The intestinal bacterial flora distribution ratio regulator of the invention may further contain other ingredients and may contain, for example, lactic acid bacteria, mineral yeasts, flavonoids, polyphenols, etc., and oral substances having an immunostimulating action.

In the case where the intestinal bacterial flora distribution ratio regulator contains these ingredients, when the intestinal bacterial flora distribution ratio regulator is solid, the content is preferably 0.1% by mass to 30% by mass and more preferably 5% by mass to 20% by mass, based on the whole intestinal bacterial flora distribution ratio regulator. When the intestinal bacterial flora distribution ratio regulator is liquid, the content is preferably 0.001% by mass to 10% by mass and more preferably 0.01% by mass to 5% by mass, based on the whole intestinal bacterial flora distribution ratio regulator.

The lactic acid bacteria such as *Bifidobacteria* are preferably those which can be orally administrated to mammals including human and exhibit a useful action in living body digestive tracts.

More specifically, desired are those which are harmless to hosts, relatively tolerant to gastric acid and bile acid, are fixative in living body intestines, produce lactic acid, and have an action of regulating the intestinal bacterial flora distribution ratio. Examples of such useful lactic acid bacteria include *Lactobacillus acidophilus, Bifidobacteria* (*Bifidobacterium longum, etc.*), *Streptococcus faecalis, Lactobacillus reuteri, Lactobacillus casei, Lactobacillus plantarum, Lactobacillus fermentum, Lactobacillus rhamnosus, Lactobacillus agilis, Lactobacillus gasseri, Bacillus mesentericus, Clostridium butyricum,* or subspecies thereof. Preferred are *Lactobacillus acidophilus, Bifidobacteria* (*Bifidobacterium longum, etc.*), and *Streptececcus faecalis.* These lactic acid bacteria can be used singly or two or more thereof can be appropriately used in combination.

Regarding these lactic acid bacteria, origins from which they are obtained are not particularly limited so long as they are living bacteria and conveniently, commercially available ones can be widely used.

In the case of setting an intake or a standard intake per day of the intestinal bacterial flora distribution ratio regulator to be used in the invention, the number of living bacteria of lactic acid bacteria in the daily amount of the intestinal bacterial flora distribution ratio regulator is preferably 10,000,000 to 100,000,000,000, more preferably 50,000,000 to 50,000,000,000, and particularly preferably 100,000,000 to 10,000,000,000.

The mineral yeasts mean yeasts containing minerals. As the minerals, there may be mentioned 16 kinds of metal elements of sodium (Na), potassium (K), chlorine (Cl), calcium (Ca), magnesium (Mg), phosphorus (P), and sulfur (S), as well as iron (Fe), zinc (Zn), copper (Cu), manganese (Mn), cobalt (Co), chromium (Cr), iodine (I), molybdenum (Mo), and selenium (Se) which are called microelements. As the mineral yeasts to be used in the invention, chromium yeasts containing chromium are preferred. The kind of the yeast is not particularly limited but Saccharomyces cerevisiae or related varieties thereof, preferably baker's yeast and beer yeast are preferred.

The chromium content of the chromium yeast is preferably 0.01 to 5 parts by mass, more preferably 0.05 to 1 part by mass, and particularly preferably 0.1 to 0.3 part by mass based on 100 parts by mass of the chromium yeast.

The content of the chromium yeast in the intestinal bacterial flora distribution ratio regulator is preferably 0.5 to 50 parts by mass, more preferably 1 to 10 parts by mass, and particularly preferably 3 to 5 parts by mass, based on the parts by mass of the intestinal bacterial flora distribution ratio regulator.

In the case of setting an intake or a standard intake per day, the amount of the chromium yeast in the daily amount of the intestinal bacterial flora distribution ratio regulator is preferably 5 to 500 mg, more preferably 10 to 100 mg, and particularly preferably 30 to 50 mg. The amount of chromium in the daily amount of the intestinal bacterial flora distribution ratio regulator is preferably 20 to 200 $\mu$g, more preferably 40 to 150 $\mu$g, and particularly preferably 60 to 100 $\mu$g.

Flovonoid is a generic name of coloring matter ingredients widely present in all organs of plants, is mainly contained in fruits and vegetables, and is particularly present in the form of glycosides in green leave and white vegetables and the peels of citrus.

In the invention, flovonoid is a generic name of coloring matter ingredients widely contained in plants and particularly means flavane derivatives richly contained in vegetables and fruits.

As flavonoids, flavonols, isoflavones, and catechins are preferred. The flavonols are known as polyphenols. The flavonoids are substances ingested into the body but are generally difficult to absorb. However, since the flovonoids are effective even in a small amount and are strong antioxidant substances, they are known to suppress the activity of carcinogens and have a blood flow facilitating action and an antithrombotic action.

In the invention, flavonoids can be obtained from respective derived products such as tea, grapes, and onion. Herein, the derived product means one extracted from at least a part of a living body. For the extraction, for example, a method for preparing the above extracts of the plant belonging to the genus *Salacia* is applied. The forms of the extract can be the same as above and, for example, may be any form such as a filtrate itself after extraction, or concentrated or diluted one or a form of dried powder thereof, or a mixture thereof.

A tea extract containing a catechin is prepared from a tea tree which is a evergreen tree of Theaceae. As the tea tree, both of *Camellia sinensis* var. *assamica* cultivated in India, Sri Lanka, and Southeast Asia and *Camellia sinensis* cultivated in China and Japan are used. For the extraction, water, an alcohol, or an aqueous alcohol is preferably used. More preferably, hot water or ethanol or an aqueous ethanol is used as the extraction solvent. Regarding the alcohol concentration of the above aqueous alcohol, one having a concentration of 30 to 90% by mass, preferably 40 to 70% by mass may be used.

As the drying method, spray drying, freeze-drying, and the like may be mentioned without limitation thereto.

The tea extract contains antioxidant substances such as polyphenols and catechins. It is preferred to contain catechin, epicatechin, gallocatechin, epigallocatechin, catechin gallate, epicatechin gallate, gallocatechin gallate, or epicatechin gallate and, particularly, it is preferred to contain epigallocatechin.

The intestinal bacterial flora distribution ratio regulator of the invention contains the tea extract in an amount of preferably 0.1 to 40% by mass, further preferably 0.5 to 35% by mass, and particularly preferably 1.0 to 30% by mass.

Moreover, flavonols, which are one of flavonoids, remove active oxygen and exhibit antioxidant actions such as suppression of sclerosis of the arteries and improvement in blood flow. Among the flavonols, resveratrol which is one of polyphenols has attracted attention as an antioxidant substance. Resveratrol is composed of a stilbene skeleton and is richly contained in the pericarp of grapes, so that it is also contained in red wine made of grapes.

In the invention, it is preferred to contain a grape extract or a wine concentrate, containing the flavonols as ingredients. The intestinal bacterial flora distribution ratio regulator of the invention preferably contains a grape extract in an amount of 0.1 to 30% by mass and further preferably contains it in an amount of 0.1 to 10% by mass.

Resveratrol has an action of burning fat, prevents sclerosis of the arteries which is a vascular disease, exhibits an anticancer action, also prevents shortening of DNA by cell division, and has a cell-life extension effect the same as in the case of caloric restriction, so that it has been realized that the compound has an excellent effect as a material for preventing lifestyle-related diseases.

The content of resveratrol in the intestinal bacterial flora distribution ratio regulator of the invention is preferably 0.0001 to 5.00% by mass and further preferably 0.001 to 2.00% by mass.

Moreover, among the flavonols, quercetin that is a polyphenol has attracted attention as an antioxidant substance. Quercetin has a flavan structure and is richly contained in the hull of onion.

For quercetin, physiological actions such as a support for absorption of vitamin C, an antioxidation action, an immune action, and the like have been reported. Furthermore, quercetin has been found to be effective for suppressing fat absorption, so that it has been realized that the compound has an excellent effect as a material for preventing lifestyle-related diseases.

The content of quercetin in the intestinal bacterial flora distribution ratio regulator is preferably 0.001 to 15% by mass, more preferably 0.05 to 10% by mass, and further preferably 0.1 to 5.0% by mass.

The intestinal bacterial flora distribution ratio regulator of the invention preferably contains particularly catechin in an amount of 1 to 50% by mass. As catechin, one derived from green tea is particularly preferred.

Moreover, the intestinal bacterial flora distribution ratio regulator of the invention preferably contains a polyphenol having a lipase activity-inhibiting effect in an amount of 2 to 80% by mass. As the polyphenol having a lipase activity-inhibiting effect, one derived from oolong tea, one derived from grapes, one derived from apple, one derived from litchi, one derived from pine bark, one derived from hesperidium, and the like are particularly preferred.

The oral substance having an immunostimulating action means one that is considered to enhance immune function, such as rose flower extracts and lotus root-derived products. The derived product means one extracted from at least a part of a living body. For the extraction, for example, a method for preparing the above extracts of the plants belonging to the genus *Salacia* is applied. The forms of the extract can be the same as above and, for example, may be any form such as a filtrate itself after extraction, or concentrated or diluted one or a form of dried powder thereof, or a mixture thereof.

<Method for Use/Formulation>

The intestinal bacterial flora distribution ratio regulator of the invention targets mammals including human and is orally administrated to the mammals. Herein, "intestinal" means "in the digestive tracts" where bacteria of *Bacteroidetes, Firmicutes, Bifidobacteriales,* and the like are always present and digestion/absorption are performed, such as human small intestine and large intestine.

The intestinal bacterial flora distribution ratio regulator of the invention may be a food (inclusive of a drink), a food material, a quasi-pharmaceutical product, a pharmaceutical, a material for a pharmaceutical, or a material for a quasi-pharmaceutical product. As the other ingredients, various carriers pharmaceutically or food-hygienically acceptable as oral administration agents, for example, excipients, lubricants, stabilizers, dispersants, binders, diluents, flavoring agents, sweeteners, seasonings, and colorants can be exemplified.

The form of the intestinal bacterial flora distribution ratio regulator of the invention is not particularly limited so long as it exerts the advantages of the invention and examples thereof include tablets, pills, granules, fine granules, masticatory drugs, capsules (those filled into hard capsules or soft capsules), liquids, chewable drugs, drinks, and the like. The form may be the other food form.

These administration forms can be prepared using conventional methods usually known in the art.

In the case of tablets, pills, and granules, if necessary, they may be formulated into conventionally coated dosage forms, such as sugar-coated tablets, gelatin-coated ones, enteric coated ones, film-coated ones, and the like. Also, tablets may be multilayered tablets such as double-layered tablets.

Into the intestinal bacterial flora distribution ratio regulator of the invention, in addition to the above, ingredients capable of oral ingestion, such as vitamins, vitamin-like substances, proteins, amino acids, oils and fats, organic acids, carbohydrates, plant-derived materials, animal-derived materials, microorganisms, food additives, and medicinal additives, can be appropriately incorporated.

By the ingestion of the intestinal bacterial flora distribution ratio regulator of the invention, weight reduction is expected through the increase of bacteria of *Bacteroidetes* and the decrease of bacteria of *Firmicutes* in the intestines. Also, by the increase of intestinal bacteria of *Bacteroidetes,* immune function is enhance through entrapment of the intestinal bacteria of *Bacteroidetes* in Peyer's patch, so that alleviation of allergy symptoms such as pollen allergy and atopy, prevention of a common cold, and prevention of carcinogenesis are expected. (Tsuda, M., Hosono, A., Yanagibashi, T., Hachimura, S., Hirayama, K., Itoh, K., Taleahashi, K., and Kaminogawa, S., Prior stimulation of antigen-presenting cells with Lactobacillus regulates excessive antigen-specific cytokine responses in vitro when compared with Bacteroides. Cytotechnology, 55, 89-101 (2007))

The intestinal bacterial flora distribution ratio regulator of the invention is safe even when taken for a long period of time and has few side effects.

Examples

The following will describe the invention with reference to Examples but the invention is not limited to the following Examples.

After root and trunk parts of *Salacia reticulate* and *Salacia oblonga* were pulverized, they were mixed each in equal weight and a liquid obtained via a hot-water extraction step at 98°C was spray-dried to obtain a *Salacia* extract powder 1. A sucrase $IC_{50}$ value of the *Salacia* extract powder 1 was 41 ($\mu$g/ml).

In addition, as Gymnema sylvestre and a mulberry leave extract, those available from K.K. Cross were used.

Moreover, as Comparative Examples, glucose, lard, and an oolong tea polymerization polyphenol (one obtained by freeze-drying Black Oolong Tea manufactured by Suntory Ltd. and extracting an oolong tea polymerization polyphenol) were used. Incidentally, with regard to Gymnema sylvestre, a mulberry leave extract, glucose, lard, and an oolong tea polymerization polyphenol, the sucrase 50% inhibitory concentration ($IC_{50}$ values) was 800 $\mu$g/mL or more in all cases. These ingredients were each dissolved in water for injection and administrated as an aqueous solution of 10 mg/ml.

(Experimental Example 1)

Eight-week-old male SD rats were divided into groups of 7 rats. Each of the following intestinal bacterial flora distribution ratio regulators was dissolved in water for injection and was ingested in an amount corresponding to 20 mg/kg/day using a stomach sonde for 1 week. The ratios of bacteria belonging to *Bacteroidetes* and *Firmicutes* to intestinal bacterial flora immediately before ingestion and immediately after ingestion were each measured by T-RFLP method (Nagashima method). However, the intestinal bacterial flora distribution ratio regulator was used in a half amount (10 mg/kg/day) in Example 1.

The ratios of *Bacteroidetes* and *Firmicutes* to intestinal bacteria flora (ratio obtained when total number of bacteria was regarded as 100%) are shown in Table 1.

[Table 1]

Table 1 Change in ratios of *Bacteroidetes, Firmicutes* in rat intestinal bacterial flora

| | Sugar absorption-suppressing substance or the like | Before ingestion | | | After ingestion | | |
|---|---|---|---|---|---|---|---|
| | | Ratio of *Bacteroidetes* [%] | Ratio of *Firmicutes* [%] | *Bacteroidetes* /*Firmicutes* | Ratio of *Bacteroidetes* [%] | Ratio of *Firmicutes* [%] | *Bacteroidetes*/ *Firmicutes* |
| Example 1 | Plant belonging to the genus *Salacia* (a half amount) | 24.8±4.3 | 56.3±6.6 | 0.440 | 34.6±2.7 | 50.3±4.8 | 0.688 |
| Example 2 | Plant belonging to the genus *Salacia* | 25.1±8.0 | 58.4±5.4 | 0.430 | 40.1±4.3 | 46.9±7.6 | 0.855 |
| Example 3 | Mulberry leave | 26.3±6.3 | 55.9±2.5 | 0.470 | 34.2±4.9 | 51.1±6.0 | 0.669 |
| Example 4 | Gymnema sylvestre | 25.5±2.2 | 58.2±3.0 | 0.438 | 31.7±5.1 | 52.4±4.3 | 0.605 |
| Comparative Example 1 | Glucose | 25.6±9.6 | 56.7±7.8 | 0.451 | 24.9±6.7 | 61.2±6.5 | 0.407 |
| Comparative Example 2 | Lard | 25.3±7.4 | 57.9±5.6 | 0.437 | 22.8±4.7 | 63.6±8.1 | 0.358 |
| Comparative Example 3 | Oolong tea polymerization polyphenol | 25.0±6.8 | 55.2±4.2 | 0.453 | 21.9±5.6 | 62.9±1.3 | 0.348 |

As a result of the Experimental Example, it was revealed that the sugar absorption-suppressing substances remarkably increase the ratio of *Bacteroidetes/Firmicutes* in the intestinal bacteria flora as compared with the other foods. According to the above Non-Patent Document 3, a proliferation effect of increasing bacteria belonging to *Bacteroidetes* is obtained by a fractooligosaccharide but the amount administrated is about 90 times as compared with the case of the invention. On the other hand, in the invention, an effect of increasing bacteria belonging to *Bacteroidetes* is obtained even in a small amount and thus the ingestion can be conveniently performed. Moreover, any foods reducing the ratio of bacteria belonging to *Firmicutes* have not been known until now but the foods are newly found.
During the test period, for all the rat groups of Examples and Comparative Examples, abnormality was not observed on chemical inspection values such as hepatic function and renal function and autopsy reports as well as on immune tissue chemical inspection results.

(Experimental Example 2)

Eight-week-old male SD rats bred with a high-fat diet (HFD32, CLEA Japan, Inc.) were divided into groups of 7 rats. Each of the following sugar absorption-suppressing substances and the like was dissolved in water for injection in an amount of 40 mg/kg/day (20 mg/kg/day only in Example 5) and ingested using a stomach sonde for 30 days. Change in body weight, fat weight, and triglyceride level in serum were measured. To a control group, only water for injection was administrated. As the sugar absorption-suppressing substances and the like, those the same as in Experimental Example 1 were used.

[Table 2]

Table 2 Change in body weight, fat weight, and triglyceride level in serum of rats

| | Sugar absorption-suppressing substance or the like | Increase in body weight [%] | Fat weight [g/100 g] | Triglyceride level in serum [mg/100 ml] |
|---|---|---|---|---|
| Comparative Example 4 | No ingestion | 143±2 | 3.68±0.22 | 163.5±4.1 |
| Comparative Example 5 | Glucose | 146±5 | 3.91±0.30 | 168.6±8.2 |
| Comparative Example 6 | Fractooligosaccharide | 145±6 | 3.56±0.19 | 170.2±10.3 |
| Example 5 | Plant belonging to the genus *Salacia* (a half amount) | 137±3[*] | 3.22±0.31[*] | 119.6±8.0[**] |
| Example 6 | Plant belonging to the genus *Salacia* | 121±4[**] | 2.69±0.14[**] | 87.9±6.8[**] |
| Example 7 | Mulberry leave | 140±1[*] | 3.05±0.09[*] | 102.1±2.7[**] |
| Example 8 | Gymnema sylvestre | 132±4[**] | 2.96±0.27[**] | 96.3±5.6[*] |
| ([*] P<0.05 [**] P<0.01 as compared with the case of no ingestion) | | | | |

As a result of the Experimental Example, the sugar absorption-suppressing substances suppressed the increase in body weight and remarkably reduced fat weight and triglyceride level in serum, thus a slimming effect being obtained. Incidentally, regarding the fractooligosaccharide in Comparative Example 6, although there was a finding that it was effective for proliferation of *Bacteroidetes,* such a slimming effect as suppression of the increase in body weight, fat weight, and triglyceride level in serum was not obtained.
During the test period, for all the rat groups of Examples and Comparative Examples, abnormality was not observed on chemical inspection values such as hepatic function and renal function and autopsy reports as well as on immune tissue chemical inspection results.

Industrial Applicability

The intestinal bacterial flora distribution ratio regulator of the invention can change the distribution ratio of the intestinal bacteria flora, specifically can increase bacteria of *Bacteroidetes* and decrease bacteria of *Firmicutes.* Thereby, slimming, improvement in physical constitution, alleviation of allergy symptoms, improvement of immune indices, and the like are expected. Moreover, the regulator is safe and has few side effects even when taken for a long period of time and can be utilized as foods including drinks and pharmaceuticals.

While the invention has been described in detail and with reference to specific embodiments thereof, it will be apparent to one skilled in the art that various changes and modifications can be made therein without departing from the spirit and scope thereof.

The present application is based on Japanese Patent Application No. 2009-104316 filed on April 22, 2009 and Japanese Patent Application No. 2009-1115563 filed on May 12, 2009, and the contents are incorporated herein by reference.

## Claims

1.    An intestinal bacterial flora distribution ratio regulator comprising at least one sugar absorption-suppressing substance.

2.    The intestinal bacterial flora distribution ratio regulator according to claim 1, wherein the sugar absorption-suppressing substance is an α-glucosidase activity-inhibiting substance.

3.    The intestinal bacterial flora distribution ratio regulator according to claim 1 or 2, wherein the sugar absorption-suppressing substance contains at least one or more selected from the group consisting of plants belonging to the genus *Salacia,* mulberry leaves, Gymnema sylvestre, and ingredients contained in extracts thereof.

4.    The intestinal bacterial flora distribution ratio regulator according to any one of claims 1 to 3, wherein the sugar absorption-suppressing substance exhibits an activity of a sucrase 50% inhibitory concentration of 10 μg/ml to 10,000 μg/ml.

5.    The intestinal bacterial flora distribution ratio regulator according to any one of claims 1 to 4, wherein the sugar absorption-suppressing substance is at least one selected from plants belonging to the genus *Salacia* selected from *Salacia reticulate, Salacia oblonga,* and *Salacia chinensis* and ingredients contained in extracts thereof.

6.    The intestinal bacterial flora distribution ratio regulator according to any one of claims 1 to 5, wherein the intestinal bacterial flora distribution ratio regulation increases a ratio of *Bacteroidetes* and decreases a ratio of *Firmicutes* in intestinal bacterial flora.

7.    A food or pharmaceutical containing the intestinal bacterial flora distribution ratio regulator according to any one of claims 1 to 6.

**INTERNATIONAL SEARCH REPORT**

| International application No. |
| --- |
| PCT/JP2010/057092 |

A. CLASSIFICATION OF SUBJECT MATTER

*A61K45/00*(2006.01)i, *A23L1/30*(2006.01)i, *A61K36/18*(2006.01)i, *A61K36/60* (2006.01)i, *A61P1/14*(2006.01)i, *A61P11/02*(2006.01)i, *A61P35/00*(2006.01)i, *A61P37/04*(2006.01)i, *A61P37/08*(2006.01)i, *A61P43/00*(2006.01)i

According to International Patent Classification (IPC) or to both national classification and IPC

B. FIELDS SEARCHED

Minimum documentation searched (classification system followed by classification symbols)

A61K45/00, A23L1/30, A61K36/18, A61K36/60, A61P1/14, A61P11/02, A61P35/00, A61P37/04, A61P37/08, A61P43/00

Documentation searched other than minimum documentation to the extent that such documents are included in the fields searched

| Jitsuyo Shinan Koho | 1922–1996 | Jitsuyo Shinan Toroku Koho | 1996–2010 |
| Kokai Jitsuyo Shinan Koho | 1971–2010 | Toroku Jitsuyo Shinan Koho | 1994–2010 |

Electronic data base consulted during the international search (name of data base and, where practicable, search terms used)

CA/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| X<br>Y | Fuminori UEDA et al., "Salasia-Zoku Shokubutsu (Salasia reticulata, Salasia oblonga) no Chonai Kankyo eno Sayo", Dai 62 Kai Japanese Society of Nutrition and Food Science Taikai Koen Yoshishu, 01 April 2008 (01.04.2008), vol.62, page 266, particularly, refer to the column of [Method], the 3rd sentence in the column of [Results and Study], etc. | 1–7<br>3–6 |
| X<br>Y | Takahiro MATSUKI et al., "Tonyobyo Kanja no Chonai Flora to α-Glucosidase Sogaiyaku ga Oyobosu Eikyo ni Tsuiteno Kento", Tonyobyo, 25 April 2005 (25.04.2005), vol.48, no.Supplement 2, page.S-266, ISSN:0021-437X, particularly, refer to lines 9 to 12 in column III-P-105, etc. | 1,2,4,6,7<br>3–6 |

☒ Further documents are listed in the continuation of Box C.　　　☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>12 May, 2010 (12.05.10) | Date of mailing of the international search report<br>25 May, 2010 (25.05.10) |
| --- | --- |
| Name and mailing address of the ISA/<br>　　Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2010/057092 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>A | Hiroshi SHIMODA et al., "Effect of an Aqueous extract of Salacia reticulata, a Useful Plant in Sri Lanka, on Postprandial Hyperglycemia in Rats and Humans", Journal of Japanese Society of Nutrition and Food Science, 1998.10, vol.51, no.5, pages 279 to 287, ISSN:0287-3516, particularly, column of English abstract on page 279, page 284, right column to page 287 | 7<br>1-6 |
| X<br>Y<br>A | Chieko MIYAHARA et al., "Kenkocha no Shocho α-Glucosidase Sogai Kassei", Kanagawa-Ken Koshu Eiseikai-Shi, 08 November 1999 (08.11.1999), vol.45, page 38, ISSN:0451-3177, particularly, column of [Results], table 1, fig. 1 | 7<br>3-6<br>1,2 |
| X<br>Y<br>A | Fuminori UEDA et al., "Development of 'Metabarrier' and 'Oxibarrier': Dietary supplements meant for lifestyle-related disease prevention", Fuji Film Kenkyu Hokoku, 21 March 2008 (21.03.2008), vol.53, pages 38 to 42, ISSN:0915-1478, CODEN:FFRDEK | 7<br>3-6<br>1,2 |
| X<br>Y<br>A | Hideaki MATSUDA et al., "Hypoglycemic Effects of Anti-Diabetic Supplemental Health Foods", Natural medicines, 20 August 2006 (20.08.2006), vol.60, no.2, pages 53 to 58, ISSN:1349-9114 | 7<br>3-6<br>1,2 |
| X<br>A | JP 2002-20302 A (Morishita Jintan Co., Ltd.), 23 January 2002 (23.01.2002), claims 2 to 4; paragraphs [0013] to [0016] (Family: none) | 7<br>1-6 |
| X<br>A | JP 2002-20308 A (Yugen Kaisha Sakamoto Yakusoen), 23 January 2002 (23.01.2002), claim 1; paragraphs [0027] to [0031] (Family: none) | 7<br>1-6 |
| X<br>A | JP 2007-31345 A (Kabushiki Kaisha Morimitsu), 08 February 2007 (08.02.2007), claims; paragraphs [0011], [0045] (Family: none) | 7<br>1-6 |
| X<br>A | JP 2003-128571 A (Matsuura Yakugyou Co., Ltd.), 08 May 2003 (08.05.2003), claim 1; paragraphs [0006] to [0010]; tables 1, 2 (Family: none) | 7<br>1-6 |

Form PCT/ISA/210 (continuation of second sheet) (July 2009)

| **INTERNATIONAL SEARCH REPORT** | International application No. |
|---|---|
| | PCT/JP2010/057092 |

<Subject of search>

In claims 1 to 7 of the present application, active ingredients are specified as "sugar absorption inhibitor" (claim 1) and "α-glucosidase activity inhibitor" (claim 2). In these claims, therefore, a substance is specified based on function, properties or the like.

Concerning the aforesaid "sugar absorption inhibitor" and "α-glucosidase activity inhibitor", examples of the individual components are cited in paragraphs [0010] to [0020] of the description. However, the description does not disclose what specific substances are included in the scopes of the aforesaid "sugar absorption inhibitor" and "α-glucosidase activity inhibitor". Even though the common technical knowledge at the point of the application is taken into consideration, the scopes thereof could not be specified. Thus, the disclosures in claims 1, 2, 4, 6 and 7 fail to satisfy the requirement of clearness as defined in Article 6 of the PCT. Nothing is disclosed under the provision of Article 5 of the PCT and supported under the provision of Article 6 of the PCT, but products comprising, as the active ingredient, a salacia powder, which is obtained by grinding roots and stems of *Salacia reticulata* and *Salacia oblonga*, mixing the former component with the equivalent weight of the latter, extracting the mixture with hot water at 98°C, and spray-drying the thus obtained liquid extract, gymnema and mulberry leaf extract.

Such being the case, the search was conducted, also taking the disclosure by the description into consideration, on the cases where the active ingredients are components contained in plants belonging to the genus *Salacia*, mulberry leaves, gymnema and extracts thereof, and on the relationship between the inhibition of α-glucosidase activity and regulation of the composition ratio of intestinal bacterial flora.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009104316 A

- JP 20091115563 B

### Non-patent literature cited in the description

- **Ley, RE. ; Turnbaugh, PJ. ; Klein, S. ; Gordon, JI.** Microbial ecology: human gut microbes associated with obesity. *Nature,* 2006, vol. 444, 1022-3
- **Turnbaugh, PJ. ; Ley, RE. ; Mahowald, MA. ; Magrini, V. ; Mardis, ER. ; Gordon, JI.** An obesity-associated gut microbiome with increased capacity for energy harvest. *Nature,* 2006, vol. 444, 1027-31

- **Nakanishi, Y ; Murashima, K. ; Ohara, H. ; Suzuki, T. ; Hayashi, H. ; Sakamoto, M. ; Fukasawa, T. ; Kubota, H. ; Hosono, A. ; Kono, T.** Increase in terminal restriction fragments of Bacteroidetes-derived 16S rRNA genes after administration of short-chain fructooligosaccharides. *Appl. Environ. Microbiol.,* 2006, vol. 72, 6271-6
- **Tsuda, M. ; Hosono, A. ; Yanagibashi, T. ; Hachimura, S. ; Hirayama, K. ; Itoh, K. ; Taleahashi, K. ; Kaminogawa, S.** Prior stimulation of antigen-presenting cells with Lactobacillus regulates excessive antigen-specific cytokine responses in vitro when compared with Bacteroides. *Cytotechnology,* 2007, vol. 55, 89-101